# EUROPÄISCHE PATENTSCHRIFT

(11) **EP 3 733 618 B1**
(45) Veröffentlichungstag und Bekanntmachung des Hinweises auf die Patenterteilung: **05.06.2024**
(21) Anmeldenummer: 20170631.4
(22) Anmeldetag: 21.04.2020
(51) Int. Cl.: C03C 3/091, C03C 10/00, C03C 8/00, C03C 4/00, C04B 41/50, C04B 41/87

(54) **NIEDRIGSCHMELZENDE GLASKERAMIK**
LOW-MELTING GLASS CERAMIC
VITROCÉRAMIQUE À FAIBLE POINT DE FUSION

(30) Priorität: 03.05.2019 EP 19172510
(43) Veröffentlichungstag der Anmeldung: 04.11.2020
(73) Patentinhaber: VITA-ZAHNFABRIK H. Rauter GmbH & Co. KG, 79713 Bad Säckingen (DE)
(72) Erfinder: Gödiker, Berit, 79713 Bad Säckingen (DE); Hackner, Michael, 79540 Lörrach (DE)
(74) Vertreter: dompatent von Kreisler Selting Werner - Partnerschaft von Patent- und Rechtsanwälten mbB

(56) Entgegenhaltungen:
- EP-A1- 3 366 260
- EP-A2- 0 468 435
- DE-A1- 10 340 597
- DE-A1-102010 035 545
- US-A- 6 121 175
- US-A1- 2010 035 215

## Beschreibung

Die vorliegende Erfindung betrifft eine Glaskeramik zur Verblendung einer dentalen Gerüststruktur, wobei sich die Glaskeramik durch einen hohen Gehalt an B₂O₃ auszeichnet, ein Verfahren zu deren Herstellung sowie deren Verwendung in der Herstellung dentaler Restaurationen.

Glaskeramiken sind Werkstoffe, die aus Glasschmelzen durch gesteuerte Kristallisation hergestellt werden. Dabei wird das Glas durch spezielle Temperaturbehandlungen in einen teils polykristallinen und teils glasigen, keramischen Zustand überführt, wodurch ein glasähnliches Produkt entsteht, dessen Eigenschaften sich aber von denen normaler Gläser unterscheiden.

Glaskeramiken finden in einer Vielzahl von technischen Gebieten Anwendung, wobei eine der bekanntesten als Kochfelder und Kochgeschirr im Haushaltsbereich liegt. Neben weiteren Anwendungen, beispielsweise als Hochleistungsreflektoren für digitale Projektoren, werden Glaskeramiken auch als Materialien bei der Herstellung von dentalen Restaurationen verwendet.

So beschreibt beispielsweise DE 197 50 794 Lithiumdisilikat-Glaskeramik-Produkte, die durch plastische Verformung unter Druck- und Wärmeeinwirkung oder spanende Bearbeitung zu geformten Dentalprodukten mit hoher Festigkeit verarbeitet werden können.

Außer für die Herstellung des Grundkörpers der dentalen Restauration können Glaskeramiken auch als Verblendmaterialien eingesetzt werden, mit deren Hilfe der natürliche Zahn aufbauend auf einer Gerüststruktur nachgebildet wird. Bei dieser Anwendung kommt es vor allem auf einen guten Verbund zwischen der Verblendung und der Gerüststruktur an, um eine stabile und langlebige dentale Restauration zu gewährleisten. Ein stabiler Verbund wird meist dadurch erreicht, dass die Eigenschaften des Verblendmaterials, insbesondere hinsichtlich der thermischen Ausdehnung, an die des Gerüstmaterials angepasst werden.

WO 2018/071408 beschreibt eine dentale Restauration mit einer Gerüststruktur auf Basis einer Lithiumdisilikat-Glaskeramik oder einer ZrOz-basierten Keramik, die wenigstens eine Verblendbeschichtung enthält, wobei die Verblendbeschichtung mit der Gerüststruktur thermisch kompatibel ist.

EP 2 405 883 offenbart eine Zusammensetzung zur Verwendung für das Befestigen einer Zahnverblendung an einer Zahnträgerstruktur, wobei die Zusammensetzung 10 bis 55 Gew.-% Wasser und 40 bis 85 Gew.-% eines glaskeramischen Materials enthält, das 55 bis 75 Gew.-% Siliciumoxid und 8 bis 22 Gew.-% Aluminiumoxid umfasst. Die Verblendstruktur ist dabei in Bezug auf eine gesinterte Verblendung mit einem Vergrößerungsfaktor von 1,12 bis 1,9 proportional vergrößert und weist einen Wärmeausdehnungskoeffizienten von 8*10⁻⁶K⁻¹ bis 15,8*10⁻⁶K⁻¹ auf.

EP 1 000 588 betrifft eine keramische Dentalrestauration bestehend aus einer Basiskeramik auf Basis einer leucithaltigen Glaskeramik, die mit einer Dentalkeramik verblendet ist, wobei die Basiskeramik als Komponenten 40 bis 95 Gew.-% SiOz und 5 bis 25 Gew.-% Al₂O₃ enthält. Die Dentalkeramik hat einen linearen Wärmeausdehnungskoeffizienten α_{(20-500°C)} von 13,5*10⁻⁶K⁻¹ bis 17,0*10⁻⁶ K⁻¹ und die Basiskeramik einen linearen Wärmeausdehnungskoeffizienten α_{(20-500°C)} von 12,5*10⁻⁶K⁻¹ bis 15,5*10⁻⁶ K⁻¹, wobei der Wärmeausdehnungskoeffizient der Basiskeramik um 1,5*10⁻⁶K⁻¹ unter dem der Verblendkeramik liegt.

DE 102010035545 A1 offenbart eine Verblendkeramik für dentale Restaurationen, wobei die Gerüstkeramik aus yttriumstabilisiertem Zirkoniumdioxid besteht. Die Verblendkeramik basiert auf Lithiumdisilikat und soll kein Leucit, Lithiummetasilicat oder beta-Spodumen enthalten. Die Verblendkeramiken weisen gemäß den Ausführungsbeispielen einen Wärmeausdehnungskoeffizienten von mehr als 9,5*10⁻⁶K⁻¹ auf.

US 6,121,175 A offenbart Alkalisilikatgläser und Apatitglaskeramiken, die Trübungsmittel, wie etwa ZnO und ZrO2 enthalten. Die beschriebenen Zusammensetzungen weisen jedoch weder eine ausreichende Temperaturwechselbeständigkeit auf noch eine geeignete Transluzenz und einen geeigneten Wärmeausdehnungskoeffizienten um sowohl Hochtemperaturwerkstoffe, wie Zirkoniumdioxid als auch niedriger schmelzende Keramiken, wie etwa Lithiumdilikat und zirkoniumoxidstabilisierte Lithiumsilikatkeramik verblenden zu können.

EP 0 544 145 betrifft einen dentalkeramischen Werkstoff zur Herstellung und Reparatur von metallkeramischem und vollkeramischem Zahnersatz mit einer Verarbeitungstemperatur unterhalb 700 °C und einem Wärmeausdehnungskoeffizienten α von 13-14*10⁻⁶ K⁻¹ zwischen 20 und 500 °C, gekennzeichnet durch die Zusammensetzung: 60 bis 65 Gew.-% SiO₂; 8,5 bis 11 Gew.-% Al₂O₃; 8 bis 12 Gew.-% KzO; 10,5 bis 12 Gew.-% NazO; 0,7 bis 2 Gew.-% CaO; 0,6 bis 2 Gew.-% BaO; 0,5 bis 2,5 Gew.-% B₂O₃; 0,1 bis 0,6 Gew.-% Sb₂O₃; 0 bis 0,5 Gew.-% CeOz; 1,2 bis 3,8 Gew.-% TiOz; 0,8 bis 1,4 Gew.-% LizO und 1,2 bis 3,8 Gew.-% F₂.

DE10340597 beschreibt Glaskeramiken für dentale Anwendungen. U

Bei der Herstellung dentaler Restauration wird die Verblendstruktur dazu verwendet, die Gerüststruktur zu verdecken und der Restauration ein möglichst natürliches Aussehen zu verleihen, so dass sie sich unauffällig in das bestehende Zahnschema einfügt. Dazu wird die Verblendstruktur auf die Gerüststruktur aufgebracht und zusammen mit dieser einer Wärmebehandlung unterzogen, die zum einen für einen festen Verbund zwischen Gerüst- und Verblendstruktur sorgen soll und zum anderen dazu dient, die optischen Eigenschaften der dentalen Restauration einzustellen. Obwohl die im Stand der Technik beschriebenen Glaskeramiken in ihrem thermischen Verhalten an die gängigen Gerüstmaterialien angepasst sind, kommt es in der Praxis immer wieder vor, dass sich durch das unterschiedliche thermische Ausdehnungsverhalten der verwendeten Materialien Risse und Sprünge in der Verblendstruktur bilden, wodurch die dentale Restauration unbrauchbar wird.

Ein weiterer Nachteil der im Stand der Technik bekannten Glaskeramiken ist deren hoher Erweichungspunkt, der eine Verarbeitung erschwert und entsprechend hohe Temperaturen beim Herstellungsprozess erfordert, wodurch sich die Zeit bis zur Fertigstellung der dentalen Restauration erheblich verlängert. Ein weiterer Nachteil der genannten hochschmelzenden Verblendmaterialien ist außerdem, dass ihre Brenntemperatur den Erweichungspunkt der zur Ausbildung der Gerüststruktur verwendeten Glaskeramiken übersteigt, insbesondere in Fällen, in denen die Gerüststruktur aus Lithiumsilikat-Glaskeramiken besteht. Der Erweichungspunkt solcher Glaskeramiken liegt je nach Zusammensetzung in der Regel zwischen 780 und 840 °C. Eine Verblendung von Gerüsten aus dieser Materialgruppe, die mittlerweile weit verbreitet im Dentalmarkt ist, ist damit nicht möglich, da sich das Gerüst beim Aufbrennen des Verblendmaterials verformen würde. Auch bei ZrOz-Gerüstmaterialien gibt es Probleme mit hochschmelzenden Verblendmaterialien. Der Erweichungspunkt von ZrOz liegt zwar weit oberhalb des Erweichungspunkts aller bekannten Verblendmaterialien, allerdings werden in der Dentalbranche häufig porös vorgesinterte ZrOz-Gerüste, eingesetzt, die mit Färbeflüssigkeiten eingefärbt sind, um die Zahnfarbe des Patienten möglichst gut zu treffen. Wenn diese eingefärbten ZrOz-Gerüste jedoch nach dem Dichtsintern nochmals auf Temperaturen oberhalb von 850 °C erhitzt werden, werden enthaltene färbende Komponenten teilweise oxidiert und es kann, je nach Zusammensetzung der verwendeten Färbeflüssigkeit, zu Abweichungen in der Farbtreue kommen.

Es ist daher Aufgabe der vorliegenden Erfindung eine Glaskeramik für die Verblendung einer Gerüststruktur zur Verfügung zu stellen, die zum einen eine gute Verarbeitbarkeit aufweist und einen stabilen Verbund mit der Gerüststruktur ausbildet. Darüber hinaus soll sich die Glaskeramik durch gute optische Eigenschaften auszeichnen, die es ermöglichen, den natürlichen Farbverlauf eines Zahns nachzubilden.

Es wurde überraschend gefunden, dass diese Aufgabe durch eine Glaskeramik gelöst wird, die einen hohen Gehalt an B₂O₃ aufweist.

Ein erster Gegenstand der vorliegenden Erfindung ist daher eine Glaskeramik zur Verblendung einer dentalen Gerüststruktur wie im unabhängigen Anspruch 1 definiert.

Die erfindungsgemäße Glaskeramik zeichnet sich insbesondere durch einen vergleichsweise niedrigen Erweichungspunkt aus, wodurch eine materialschonende Verarbeitung möglich ist, was insbesondere im Hinblick auf die Temperatursensibilität der Materialien von Bedeutung ist, die für die Gerüststrukturen verwendet werden. Es wurde überraschend gefunden, dass dank der mit der niedrigen Erweichungstemperatur verbundenen niedrigen Verarbeitungstemperatur der Glaskeramik die sonst üblicherweise auftretende Verformung der Gerüststruktur reduziert und der Verbund zwischen Verblendkeramik und Gerüststruktur verbessert werden kann. Im Rahmen der vorliegenden Erfindung hat es sich als besonders vorteilhaft erwiesen, wenn der Erweichungspunkt der Glaskeramik nicht über 800 °C liegt. Daher ist eine Ausführungsform der erfindungsgemäßen Glaskeramik bevorzugt, in der die Glaskeramik einen Erweichungspunkt von weniger als 790 °C aufweist, vorzugsweise weniger als 780 °C, besonders bevorzugt 730 bis 770 °C. Der Erweichungspunkt kann dabei mittels Erhitzungsmikroskopie bestimmt werden, wie beispielsweise in DIN 51730 dargelegt.

Die Eigenschaften der erfindungsgemäßen Glaskeramik sind insbesondere auf die Eigenschaften der zur Herstellung der Gerüststruktur verwendeten Materialien abgestimmt, um einen stabilen Verbund zwischen Gerüststruktur und Verblendkeramik zu gewährleisten. Zur Einstellung insbesondere der thermischen Eigenschaften der Glaskeramik kann diese weitere Komponenten enthalten.

In einer bevorzugten Ausführungsform weist die erfindungsgemäße Glaskeramik weiterhin K₂O in einer Menge von 7 bis 9 Gew.-% auf, jeweils bezogen auf das Gesamtgewicht der Glaskeramik. Das Vorliegen von K₂O in den erfindungsgemäßen Mengen begünstigt die Ausbildung von Leucitkristallen in den erfindungsgemäßen Glaskeramiken. Das Vorliegen von Leucitkristallen in den erfindungsgemäßen Glaskeramiken verstärkt die physikalischen Eigenschaften sowie die Säurebeständigkeit. Die Glaskeramiken weisen (K[AlSi₂O₆]) auf. In einer weiteren Ausgestaltung der vorliegenden Erfindung weist die Glaskeramik eine Kristallphase auf, die vorzugsweise als Hauptbestandteil Leucit aufweist.

In einer Ausgestaltung weist die Glaskeramik Leucit in einer Menge von 0,1 bis 10 Gew.-%, vorzugsweise in einer Menge von 0,2 bis 5 Gew.-% oder 1 bis 4 Gew.-% auf. Der Leucitgehalt kann mittels Rietveld-Methode bestimmt werden.

In einer weiterhin bevorzugten Ausführungsform weist die Glaskeramik Al₂O₃ in einer von 3 bis 11 Gew.-%, vorzugsweise 5 bis 9 Gew.-% auf. Al₂O₃ begünstigt ebenfalls den Erhalt von Leucitkristallen in den erfindungsgemäßen Glaskeramiken.

Ebenfalls als vorteilhaft hat sich die Anwesenheit von Na₂O erwiesen. Daher ist eine Ausführungsform bevorzugt, in der die erfindungsgemäße Glaskeramik Na₂O in einer Menge von 4 bis 11 Gew.-%, vorzugsweise 5 bis 7 Gew.-%, enthält, jeweils bezogen auf das Gesamtgewicht der Glaskeramik.

Das Zusammenspiel aus B₂O₃ und K₂O in den erfindungsgemäß einzusetzenden Mengen hat sich als besonders positiv für die Strukturverfestigung der Glaskeramik erwiesen.

Im Gegensatz zu anderen Alkalioxiden hat sich die Anwesenheit von LizO in der Glaskeramik als wenig vorteilhaft erwiesen, insbesondere im Hinblick auf die Verbundhaftung mit der Gerüststruktur. Daher ist der Anteil von Li₂O in der Glaskeramik weniger als 3 Gew.-%, vorzugsweise weniger als 2 Gew.-%, besonders bevorzugt 0,1 bis 1,5 Gew.-% jeweils bezogen auf das Gesamtgewicht der Glaskeramik. Der niedrige Gehalt an LizO führt zudem zur Vermeidung der Ausbildung von Lithiumsilikatkristallen in den erfindungsgemäßen Glaskeramiken.

Das Vorliegen von Lithiumsilikatkristallen in den erfindungsgemäßen Glaskristallen ist bevorzugt niedrig zu halten um die physikalisch und chemisch vorteilhaften Eigenschaften nicht negativ zu beeinflussen.

In einer weiteren vorteilhaften Ausgestaltung der vorliegenden Erfindung umfasst die Menge der Lithiumsilikatkristalle weniger als 1 Gew.-%, insbesondere weniger als 0,1 Gew.-% und speziell weniger als 0,01 Gew.-%, bezogen auf das Gesamtgewicht der Glaskeramik. Speziell bevorzugt ist eine Glaskeramik, die im Wesentlichen frei von Lithiumdisilikat und/oder Lithiummetasilikat ist. Im Wesentlichen frei im Sinne der vorliegenden Erfindung ist ein Anteil unterhalb von 0,01 Gew.-%, bevorzugt jedoch gänzlich frei. Die Bestimmung der Lithiumsilikatkristalle in einer Glaskeramik kann mittels Rietveld-Methode bestimmt werden.

In einer besonders bevorzugten Ausführungsform umfasst die erfindungsgemäße Glaskeramik die folgenden Komponenten:
- 60 bis 75 Gew.-%, vorzugsweise 65 bis 70 Gew.-% SiO₂;
- 6 bis 12 Gew.-%, vorzugsweise 7 bis 10 Gew.-% B₂O₃;
- 6 bis 12 Gew.-%, vorzugsweise 7 bis 9 Gew.-% KzO;
- 3 bis 11 Gew.-%, vorzugsweise 5 bis 9 Gew.-% Al₂O₃;
- 4 bis 11 Gew.-%, vorzugsweise 5 bis 7 Gew.-% NazO;
- 0 bis 3 Gew.-%, vorzugsweise 0,1 bis 1,5 Gew.-% LizO,
wobei sich die Angaben in Gew.-% jeweils auf das Gesamtgewicht der Glaskeramik beziehen.

Zur Verbesserung der Transluzenz hat sich als vorteilhaft gezeigt, wenn die erfindungsgemäßen Glaskeramiken die Komponenten ausgewählt aus der Gruppe bestehend aus BeO, TiO₂, ZnO, BaO, P₂O₅, PbO, CaF₂ und NaF in nur geringen Mengen, vorzugsweise unterhalb von 1,5 Gew.-%, insbesondere unterhalb von 1,0 Gew.-% und im Speziellen unterhalb von 0.5 Gew.-% oder unterhalb von 0.1 Gew.-% aufweisen. Bevorzugt sind auch Glaskeramiken, die im Wesentlichen frei von den zuvor genannten Komponenten sind.

In einer weiterhin bevorzugten Ausführungsform weist die erfindungsgemäße Glaskeramik weniger als 0,5 Gew.-% ZnO, vorzugsweise weniger als 0,1 Gew.-% ZnO auf, jeweils bezogen auf das Gesamtgewicht der Glaskeramik. Speziell bevorzugt ist eine Ausführungsform der vorliegenden Erfindung, bei der die erfindungsgemäße Glaskeramik im Wesentlichen frei von ZnO ist.

Weiterhin bevorzugt ist eine Ausführungsform, in der die erfindungsgemäße Glaskeramik weniger als 15 Gew.-% ZrOz, vorzugsweise weniger als 5 Gew.-% ZrOz aufweist, besonders bevorzugt weniger als 1 Gew.-% ZrOz aufweist jeweils bezogen auf das Gesamtgewicht der Glaskeramik. Speziell bevorzugt ist eine Ausführungsform der vorliegenden Erfindung, bei der die erfindungsgemäße Glaskeramik im Wesentlichen frei von ZrOz ist.

Weiterhin bevorzugt ist eine Ausführungsform, in der die erfindungsgemäße Glaskeramik weniger als 0,5 Gew.-% BaO, vorzugsweise weniger als 0,1 Gew.-% BaO aufweist, jeweils bezogen auf das Gesamtgewicht der Glaskeramik. Speziell bevorzugt ist eine Ausführungsform der vorliegenden Erfindung, bei der die erfindungsgemäße Glaskeramik im Wesentlichen frei von BaO ist.

In einer weiteren Ausgestaltung der vorliegenden Erfindung liegt die Summe der Bestandteile bestehend aus BaO und ZnO unterhalb von 1,0 Gew.-%, bevorzugt unterhalb von 0,5 Gew.-% und im speziellen unterhalb von 0,1 Gew.-%, wobei die Gewichtsangaben jeweils bezogen auf das Gesamtgewicht der Glaskeramik bezogen ist.

Dem Fachmann auf dem Gebiet der Glaskeramik ist bekannt, dass die Angabe der Komponenten der Glaskeramik in Form ihrer Oxide eine übliche Methode zur Beschreibung einer Glaskeramik ist. Nichtsdestotrotz sei an dieser Stelle klargestellt, dass die erfindungsgemäße Glaskeramik vorzugsweise aus einer Ausgangsmischung erhalten wird, die die Komponenten der Glaskeramik in Form ihrer Oxide enthält.

In einer bevorzugten Ausgestaltung der vorliegenden Erfindung weist die erfindungsgemäße Glaskeramik eine Transluzenz oberhalb von 75 %, insbesondere oberhalb von 80% auf.

Zur Bestimmung derTransluzenz wurden Plättchen (1,5 g, Durchmesser > 14 mm) aus Keramikpulver gestanzt und testweise im Dentalofen gebrannt. Anschließend wurden die Plättchen mittels Schleifen auf eine Enddicke von 1,00 ± 0,01 mm gebracht und beidseitig auf Hochglanz poliert. Die Messung erfolgte mit einem handelsüblichen Photospektrometer im sichtbaren Bereich des Lichtspektrums (360 bis 740 nm).

Die erfindungsgemäße Glaskeramik zeichnet sich dadurch aus, dass sie einen stabilen Verbund mit der Gerüststruktur ausbildet und so ein Abplatzen oder eine Beschädigung unter Belastung, beispielsweise während des Kauvorgangs, auch bei geringen Verblenddicken vermieden wird. Dieser vorteilhafte Verbund wird insbesondere dadurch erreicht, dass der Wärmeausdehnungskoeffizient WAK der Glaskeramik auf das Material der Gerüststruktur abgestimmt ist. Dabei darf der WAK der erfindungsgemäßen Glaskeramik den WAK des Gerüsts keinesfalls überschreiten. Als besonders vorteilhaft hat es sich erwiesen, wenn der WAK der erfindungsgemäßen Glaskeramik 0,1 bis 2,5*10⁻⁶K⁻¹ unterhalb des WAK der Gerüststruktur liegt. Daher ist eine Ausführungsform bevorzugt, in der die Glaskeramik einen Wärmeausdehnungskoeffizienten WAK von weniger als 9,5*10⁻⁶K⁻¹, bevorzugt von 9,3 *10⁻⁶K⁻¹ oder weniger, besonders bevorzugt von 8,3 *10⁻⁶K⁻¹ bis 9,3 *10⁻⁶K⁻¹ oder von 8,3 *10⁻⁶K⁻¹ bis 9,0 *10⁻⁶K⁻¹ aufweist, bestimmt mittels Dilatometer. Der Wärmeausdehnungskoeffizient wurde bei einer Temperatur unterhalb von 800 °C, insbesondere im Bereich von 100 bis 400 °C, bestimmt.

Die erfindungsgemäße Glaskeramik zeichnet sich weiterhin durch eine hohe chemische Beständigkeit aus, was sie besonders für die Verwendung im Dentalbereich geeignet macht. Es wurde überraschend gefunden, dass die erfindungsgemäße Glaskeramik trotz des hohen Gehalts an B₂O₃ eine geringe Löslichkeit, insbesondere in sauren Milieus aufweist. Daher ist eine Ausführungsform bevorzugt, in der die erfindungsgemäße Glaskeramik eine Löslichkeit von weniger als 20 µg/cm², vorzugsweise weniger als 10 µm/cm², besonders bevorzugt 1 bis 5 µg/cm² aufweist. Die Löslichkeit kann dabei beispielsweise gemäß DIN ISO 6872 bestimmt werden.

Die Glaskeramik wird vorzugsweise in Form eines Pulvers zur Verfügung gestellt, das mit Hilfe eines flüssigen Mediums zu einer Paste verarbeitet wird, die dann auf die Gerüststruktur aufgebracht wird. Dabei hat sich überraschend gezeigt, dass die Verbundhaftung zwischen Glaskeramik und Gerüststruktur erhöht werden kann, wenn die Glaskeramik in Form eines Pulvers mit einer ausgewählten Partikelgrößenverteilung eingesetzt wird. Daher ist eine Ausführungsform bevorzugt, in der die Glaskeramik in Form eines Pulvers vorliegt, das eine Partikelgrößenverteilung D50 von 15 bis 35 µm, vorzugsweise 20 bis 25 µm aufweist, bestimmt mittels Lasergranulometer. Weiterhin bevorzugt weist die erfindungsgemäße Glaskeramik in Form eines Pulvers eine Partikelgrößenverteilung D90 von 50 bis 80 µm, vorzugsweise 60 bis 75 µm auf, bestimmt mittels Lasergranulometer. In einer bevorzugten Ausführungsform weist die erfindungsgemäße Glaskeramik in Form eines Pulvers eine Partikelgrößenverteilung D10 von 2 bis 10 µm, vorzugsweise 3 bis 5 µm auf, bestimmt mittels Lasergranulometer.

Insbesondere die Einstellung der Partikelgrößenverteilung der erfindungsgemäßen Glaskeramik hat sich als ein wichtiger Faktor bei der Ausbildung eines stabilen Verbunds zwischen der Gerüststruktur und der Glaskeramik erwiesen. Die Partikelgrößenverteilung kann beispielsweise dadurch erreicht werden, dass die Glaskeramik während ihrer Herstellung mehreren Mahl- und Schmelzvorgängen unterzogen wird. Daher ist ein weiterer Gegenstand der vorliegenden Erfindung ein Verfahren zur Herstellung der erfindungsgemäßen Glaskeramik, das die folgenden Schritte umfasst:
a) Herstellen eines Ausgangsglases durch Schmelzen der Grundkomponenten und Abschrecken der Schmelze in Wasser,
b) Aufmahlen des Glases aus Schritt a) unter Erhalt eines Pulvers;
c) Verpressen des Pulvers aus Schritt b) unter Erhalt eines Rohlings;
d) Wärmebehandeln des Rohlings unter Erhalt einer Glaskeramik; und
e) Aufmahlen des Rohlings aus Schritt d) unter Erhalt eines Pulvers.

Vorzugsweise erfolgt die Herstellung des Glases in Schritt a) des erfindungsgemäßen Verfahrens ausgehend von einer Ausgangsmischung, die die Komponenten der Glaskeramik in Form ihrer Oxide enthält.

In einer bevorzugten Ausgestaltung der vorliegenden Erfindung erfolgt die Herstellung des Ausgangsglases in Schritt a) zweistufig. Hierzu wird in einem Schritt a-i) zunächst ein Leucit-Teilfrittenpulver hergestellt, dass geeignet ist Leucitkristalle auszubilden. Die Herstellung des Leucit-Teilfrittenpulvers erfolgt vorzugsweise durch Schmelzen der Grundkomponenten für die Leucit-Teilfritte und anschließendes Abschrecken in Wasser und ggf. Mahlen der so erhaltenen Leucit-Teilfritte. Vorzugsweise weist das Leucit-Teilfrittenpulver die folgende Zusammensetzung auf:
50 bis 60 Gew.-%, vorzugsweise 52 bis 58 Gew.-% SiOz,
12 bis 18 Gew.-%, vorzugsweise 13 bis 17 Gew.-% KzO,
12 bis 18 Gew.-%, vorzugsweise 13 bis 17 Gew.-% Al₂O₃,
gegebenenfalls 4 bis 10 Gew.-%, vorzugsweise 5 bis 9 Gew.-% NazO,
gegebenenfalls 1,5 bis 6 Gew.-%, vorzugsweise 2 bis 5 Gew.-% B₂O₃,
gegebenenfalls 0,5 bis 3 Gew.-%, vorzugsweise 1,0 bis 2,5 Gew.-% CaO und
gegebenenfalls 0 bis 2 Gew.-%, vorzugsweise 0,5 bis 1,6 Gew.-% LizO.

In einem Schritt a-ii) wird weiterhin Glas-Teilfrittenpulver hergestellt, welches amorph ist. Die Herstellung des Glas-Teilfrittenpulvers erfolgt vorzugsweise durch Schmelzen der Grundkomponenten für die Glas-Teilfritte und anschließendes Abschrecken in Wasser und ggf. Mahlen der so erhaltenen Glas-Teilfritte. Die Zusammensetzung der Glasteilfritte wird so gewählt, dass sie beim Vermischen mit der Leucit-Teilfritte aus Schritt a-i) die Zusammensetzung der erfindungsgemäßen Glaskeramik ergibt. Vorzugsweise weist das Glas-Teilfrittenpulver die folgende Zusammensetzung auf:
65 bis 80 Gew.-%, vorzugsweise 68 bis 75 Gew.-% SiOz,
4 bis 10 Gew.-%, vorzugsweise 5 bis 9 Gew.-% KzO,
2 bis 10 Gew.-%, vorzugsweise 3 bis 9 Gew.-% Al₂O₃,
gegebenenfalls 4 bis 10 Gew.-%, vorzugsweise 5 bis 8 Gew.-% NazO,
gegebenenfalls 5 bis 12 Gew.-%, vorzugsweise 6,5 bis 10 Gew.-% B₂O₃,
gegebenenfalls 0,5 bis 3 Gew.-%, vorzugsweise 1,0 bis 2,5 Gew.-% CaO und
gegebenenfalls 0 bis 2 Gew.-%, vorzugsweise 0,5 bis 1,6 Gew.-% LizO.

Die aus Schritt a-i) und Schritt a-ii) erhaltenen Pulver können anschließend in einem geeigneten Mischungsverhältnis gemischt werden. Das Mischungsverhältnis des Leucit-Teilfrittenpulvers zu dem Glas-Teilfrittenpulver wird so gewählt, dass die Zusammensetzungen der erfindungsgemäßen Glaskeramiken erhalten werden. Üblicherweise beträgt das Gewichtsverhältnis von Leucit-Teilfrittenpulver zu Glas-Teilfrittenpulver 1:20 bis 1:1, vorzugsweise 1:10 bis 1:2.

Die Pulvermischungen werden anschließend gemäß den Schritten c) bis e) weiterverarbeitet. Durch die Wärmebehandlung in Schritt d) kommt es bevorzugt zur Ausbildung von Leucit-Kristallen.

Die Wärmebehandlung zur Ausbildung der Glaskeramik in Schritt d) des erfindungsgemäßen Verfahrens wird vorzugsweise bei einer Temperatur von 800 bis 900 °C, bevorzugt 820 bis 880 °C, besonders bevorzugt 830 bis 850 °C. durchgeführt.

Die erfindungsgemäße Glaskeramik ist insbesondere für die Herstellung von Verblendstrukturen geeignet, die auf Gerüststrukturen aufgebracht werden, um eine dentale Restauration zu erhalten. Daher ist ein weiterer Gegenstand der vorliegenden Erfindung die Verwendung der erfindungsgemäßen Glaskeramik zur Verblendung einer Gerüststruktur, vorzugsweise einer keramischen Gerüststruktur auf Basis von Lithiumdisilikat oder ZrOz.

Um einen stabilen Verbund zwischen der Verblendstruktur und der Gerüststruktur zu erreichen, hat es sich als vorteilhaft erwiesen, wenn die Verblendstruktur und die Gerüststruktur einen ähnlichen Wärmeausdehnungskoeffizienten aufweisen. Daher ist eine Ausführungsform bevorzugt, in der die Differenz zwischen dem Wärmeausdehnungskoeffizienten der Verblendstruktur WAK_{VK} und dem Wärmeausdehnungskoeffizienten der Gerüststruktur WAK_{G} nicht mehr als 2,5*10⁻⁶K⁻¹, vorzugsweise weniger als 1,5*10⁻⁶K⁻¹, besonders bevorzugt weniger als 1,0*10⁻⁶K⁻¹, wobei die Gerüststruktur einen höheren WAK als die Verblendstruktur aufweist und der Wärmeausdehnungskoeffizient jeweils mittels Dilatometer bestimmbar ist.

Ein weiterer Gegenstand der vorliegenden Erfindung ist eine dentale Restauration umfassend eine Gerüststruktur und eine Verblendstruktur, wobei es sich bei der Verblendstruktur um eine Glaskeramik gemäß der vorliegenden Erfindung handelt. Bei der Gerüststruktur handelt es sich vorzugsweise um eine keramische Gerüststruktur auf Basis von Lithiumdisilikat oder ZrOz. Die Gerüststruktur kann dabei so ausgebildet sein, dass sie den natürlichen Farbverlauf eines Zahns nachbildet. Auf diese Weise wird ein aufwendiges Einfärben der Verblendstruktur vermieden. Das Einfärben der Gerüststruktur kann dabei beispielsweise durch das Einbringen färbender Oxide oder mittels Färbelösungen erfolgen. Hier hat sich überraschend gezeigt, dass die optischen Eigenschaften des Gerüstes durch das Aufbrennen der Verblendstruktur nicht beeinflusst werden. Daher ist eine Ausführungsform bevorzugt, bei der die Gerüststruktur einen Farbverlauf aufweist. Alternativ ist eine Ausführungsform bevorzugt, bei der die Gerüststruktur gefärbt ist.

Es wurde überraschend gefunden, dass mit Hilfe der erfindungsgemäßen Glaskeramik ein stabiler Verbund zwischen Verblendstruktur und Gerüststruktur auch bei geringen Dicken der Verblendstruktur erreicht werden kann. Daher ist eine Ausführungsform bevorzugt, in der die Dicke der Verblendstruktur 0,2 bis 3 mm, vorzugsweise 0,5 bis 1,5 mm beträgt.

Um diese geringen Dicken zu erreichen, hat es sich als vorteilhaft erwiesen, wenn die Verblendstruktur in Form einer Paste auf die Gerüststruktur aufgebracht wird. Daher ist ein weiterer Gegenstand der vorliegenden Erfindung eine Paste umfassend ein flüssiges Medium und die erfindungsgemäße Glaskeramik. Vorzugsweise handelt es sich bei dem flüssigen Medium um Wasser, das gegebenenfalls weitere Komponenten enthalten kann.

Ein weiterer Gegenstand der vorliegenden Erfindung ist ein Verfahren zur Herstellung einer dentalen Restauration, bei dem eine Glaskeramik gemäß der vorliegenden Erfindung oder eine Paste gemäß der vorliegenden Erfindung auf eine Gerüststruktur aufgebracht wird. In einer bevorzugten Ausführungsform handelt es sich bei der Gerüststruktur um eine keramische Gerüststruktur, insbesondere auf Basis von Lithiumdisilikat oder ZrOz.

Die vorliegende Erfindung wird anhand der folgenden Beispiele näher erläutert, wobei diese keinesfalls als Einschränkung des Erfindungsgedankens zu verstehen sind.

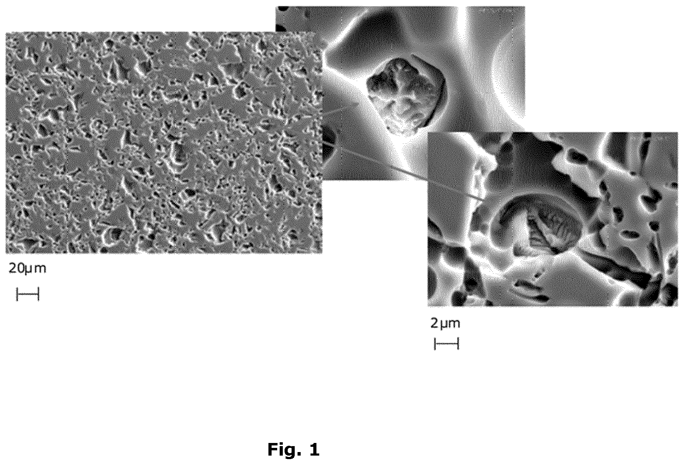

Figure 1 zeigt eine REM-Aufnahme einer erfindungsgemäßen Glaskeramik gemäß Beispielteil II. Die Glaskeramik wurde für 90 s mit 5%-iger HF behandelt. Die geätzten Stellen zeigen die Positionen an, an denen Leucit vorlag.

### Beispiele:

### Beispielteil I:

Die erfindungsgemäße Glaskeramik wurde als Verblendmaterial auf verschiedene Gerüststrukturen aufgebracht und die Temperaturwechselbeständigkeit gemäß DIN EN ISO 9693-2:2016-07 getestet. Dazu wurden die verblendeten Gerüststrukturen abwechselnd im Ofen erwärmt und dann in Eiswasser abgeschreckt, wobei die Ofentemperatur nach jedem Abschrecken um 15 °C erhöht wurde. Die Haltezeit im Ofen betrug jeweils 30 Minuten und die Probenkörper wurden nach jedem Abschrecken auf Rissbildungen und Abplatzungen untersucht. Die Ergebnisse sind in den folgenden Tabellen zusammengefasst.

Die erfindungsgemäßen Glaskeramiken wiesen dabei einen Anteil von B₂O₃ von 8 Gew.-% auf. Als Vergleich wurden herkömmliche Glaskeramiken mit einem Anteil an B₂O₃ von 1 Gew.-% (Vgl. 1) beziehungsweise 5 Gew.-% (Vgl. 2) herangezogen.

**Tabelle 1: Temperaturwechselbeständigkeit (DIN EN ISO 9693-2:2016-07) auf Lithiumdisilikat als Gerüstmaterial**

| | erfindungsgemäße Glaskeramik | Vgl. 1 | Vgl. 2 |
|---|---|---|---|
| Sprünge bei 105 °C | keine | keine | keine |
| Sprünge bei 120 °C | keine | keine | keine |
| Sprünge bei 135 °C | keine | keine | ja |
| Sprünge bei 150 °C | keine | keine | ja |
| Sprünge bei 165 °C | keine | ja | ja |
| unbeschädigte Probenkörper | 7/7 | 5/7 | 0/7 |

**Tabelle 2: Temperaturwechselbeständigkeit ZrOz-Gerüsten**

| | erfindungsgemäße Glaskeramik | Vgl. 1 | Vgl. 2 |
|---|---|---|---|
| Sprünge bei 105 °C | keine | keine | keine |
| Sprünge bei 120 °C | keine | keine | keine |
| Sprünge bei 135 °C | keine | ja | ja |
| Sprünge bei 150 °C | keine | keine | ja |
| Sprünge bei 165 °C | ja | ja | - |
| unbeschädigte Probenkörper | 5/7 | 1/7 | 0/7 |

Weiterhin wurde die Verbundhaftung der Verblendmaterialien mit Gerüstmaterialien auf Basis von ZrOz mittels des Ablöse-/Rissbeginn-Prüfung (DIN EN ISO 9693-2:2016-07) bestimmt. Die Ergebnisse sind in Tabelle 3 zusammengefasst.

**Tabelle 3:**

| | Mittelwert [MPa] | Standardabweichung [MPa] |
|---|---|---|
| erfindungsgemäße Glaskeramik | 45,5 | 5,9 |
| Vgl. 1 | 32,4 | 6,3 |
| Vgl. 2 | 36,8 | 4,8 |

Wie aus der Tabelle ersichtlich, weist die erfindungsgemäße Glaskeramik eine hervorragende Verbundhaftung auf. Der Erweichungspunkt der erfindungsgemäßen Glaskeramik wurde mittels Erhitzungsmikroskopie auf 762 °C bestimmt.

### Beispielteil II:

Bei den Beispielzusammensetzungen MU/034/18, MU/035/18 und MU/036/18 handelt es sich um Endfritten, die sich jeweils aus 2 Teilfritten zusammensetzen. Jeweils eine Teilfritte ist eine Leucitfritte mit hohem WAK. In der Leucitfritte kristallisieren nach Wärmebehandlung Leucitkristalle. Die andere Teilfritte ist jeweils eine Glasfritte, mit niedrigem WAK. Die Glasfritte bleibt auch nach Wärmebehandlung amorph und bildet keine Kristalle aus.

Glasfritte und Leucitfritte werden in einem Verhältnis gemischt, bei welchem nach Wärmebehandlung ein bestimmter Anteil ein Leucit kristallisiert, durch den wiederum der gewünschte WAK der Endfritte eingestellt wird.

Die Herstellung der Endfritten erfolgt indem jeweils unabhängig voneinander die Komponenten der Glasfritte und der Leucitfritte gemischt, aufgeschmolzen und anschließend in Wasser abgeschreckt werden. Die jeweils abgeschreckten Pulver werden zu Pulver gemahlen. Die so erhaltenen Teilfritten-Pulver von Leucitfritte und Glasfritte werden anschließend in den in Tabelle 4 aufgeführten Gew.-%-Anteilen gemischt. Anschließend wird Pulvergemisch zu einem Rohling verpresst und der Rohling einer Wärmebehandlung unterzogen unter Erhalt der Glaskeramik. Es bilden sich bei der Wärmebehandlung Leucitkristalle aus. Der wärmebehandelte Rohling wird anschließend vermahlen unter Erhalt des Endfrittenpulvers.

Folgende 3 Endfritten und 4 Teilfritten werden nachfolgend beschrieben und sind hier tabellarisch erfasst:

**Tabelle 4**

| **Endfritten** | **Teilfritten** | |
|---|---|---|
| | Leucitfritte (und deren Gew.-%-Anteil) | Glasfritte (und deren Gew.-%-Anteil) |
| **MU/034/18** | SD (27,5%) | HAK109 (77,5%) |
| **MU/035/18** | HAK105 (10%) | HAK108 (90%) |
| **MU/036/18** | HAK105 (22,5%) | HAK109 (77,5%) |

### Chemische Zusammensetzung:

Die chemische Zusammensetzung aller Beispiele ermittelt. Die Bestimmung der Anteile der folgenden Oxide wurden mittels Röntgenfluoreszenz bestimmt: SiOz, MgO, NazO, Fe₂O₃, MnO, TiOz, P₂O₅, CaO, KzO, Al₂O₃, BaO, ZnO, ZrOz, SnOz, Cr₂O₃, CoO, NiO, Sb₂O₃, La₂O₃, CeOz. Die Anteile folgender Oxide wurden mittels Atomabsorptionsspektrometrie nach KOH-Aufschluss ermittelt: B₂O₃, LizO. Der Glühverlust wurde nach DIN EN ISO 26845:2008-06 Abschnitt 9 (1050 ± 50°C/1h) ermittelt. In der unten aufgeführten Tabelle 5 wurden nur Bestandteile berücksichtigt, die laut Analyse-Ergebnis anteilig ≥ 0,01 wt.% liegen.

Da die chemische Zusammensetzung aller Teilfritten vorliegt, ist es möglich die chemische Zusammensetzung der Endfritten aus den Teilfritten zu berechnen (Tabelle 6).

### Materialeigenschaften:

Zur Bestimmung der Brenntemperatur wurden Plättchen (0,7g) aus Keramikpulver gestanzt und testweise im Dentalofen gebrannt.

Folgende Materialeigenschaften wurden gemäß ISO 6872 bestimmt: Wärmeausdehnungskoeffizient (WAK), Säurelöslichkeit und Biegefestigkeit. Gemäß ISO 6872 wird die erfindungsgemäße Glaskeramik als Typ I Keramik sowie als Klasse 1b eingeordnet. Entsprechend gelten die Prüfanweisungen und Mindestanforderung für diesen Typ bzw. diese Klasse. Da Typ I Keramiken durch das mehrfache Brennen beeinflusst werden können, wird der WAK gemäß Norm nach zweifachem sowie vierfachem Brand des Probekörpers bestimmt, wobei eine möglichst geringe Differenz zwischen beiden Messergebnissen erreicht werden sollte.

Folgende Materialeigenschaften wurden gemäß ISO 9693 bestimmt: Temperaturwechselbeständigkeit und Ablöse-/Rissbeginn-Prüfung. Die Temperaturwechselbeständigkeit ist für alle Gerüstmaterialien möglich, die im WAK zur erfindungsgemäßen Keramik passen. Die Ablöse-/Rissbeginn-Prüfung ist nur für Zirkonoxidgerüste freigegeben.

**Tabelle 5: Chemische Zusammensetzungen gemäß Analyseergebnis:**

| **wt. %** | **Teilfritten** | | | | **Endfritten** | |
|---|---|---|---|---|---|---|
| | **Leucitfritten** | | **Glasfritten** | | | |
| | **SD** | **HAK105** | **HAK10 8** | **HAK1 09** | **MU/03 4/18** | **MU/03 6/18** |
| SiO₂ | 54,41 | 55,98 | 70,70 | 71,62 | 66,21 | 67,46 |
| K₂O | 14,23 | 16,04 | 7,69 | 6,13 | 8,16 | 8,30 |
| Al₂O₃ | 15,51 | 14,03 | 8,85 | 4,24 | 7,21 | 6,32 |
| Na₂O | 8,68 | 6,16 | 6,45 | 5,65 | 6,29 | 5,50 |
| B₂O₃ | 4,76 | 3,12 | 2,59 | 8,34 | 8,31 | 7,84 |
| CaO | 1,28 | 1,98 | 1,34 | 2,13 | 1,92 | 2,10 |
| Li₂O | <0,01 | 1,46 | 1,68 | 0,90 | 0,78 | 1,15 |
| TiO₂ | 0,37 | 0,01 | 0,04 | 0,02 | 0,11 | 0,01 |
| P₂O₅ | 0,02 | <0,01 | <0,01 | <0,01 | 0,02 | <0,01 |
| Fe₂O₃ | <0,01 | 0,03 | 0,02 | 0,02 | 0,03 | 0,02 |
| MgO | <0,01 | 0,02 | 0,02 | 0,04 | 0,02 | 0,03 |
| ZrOz | <0,01 | 0,01 | 0,02 | 0,03 | <0,01 | 0,02 |
| BaO | 0,01 | 0,08 | <0,01 | <0,01 | 0,06 | 0,02 |
| Glühverl ust | 0,30 | 0,27 | 0,29 | 0,07 | 0,11 | 0,14 |
| Summe | 99,57 | 99,19 | 99,68 | 99,19 | 99,23 | 98,91 |

**Tabelle 6: Chemische Zusammensetzungen gemäß Berechnung aus den Analyseergebnissen der Teilfritten:**

| **wt.%** | **Endfritten** | | |
|---|---|---|---|
| | **MU/034/18** | **MU/035/18 (Vergl.)** | **MU/036/18** |
| SiO₂ | 66,89 | 69,23 | 68,10 |
| K₂O | 8,36 | 8,53 | 8,36 |
| Al₂O₃ | 7,34 | 9,37 | 6,44 |
| Na₂O | 6,48 | 6,42 | 5,76 |
| B₂O₃ | 7,35 | 2,64 | 7,17 |
| CaO | 1,90 | 1,40 | 2,10 |
| Li₂O | 0,65 | 1,66 | 1,03 |
| TiO₂ | 0,12 | 0,04 | 0,02 |
| P₂O₅ | 0,01 | 0,00 | 0,00 |
| Fe₂O₃ | 0,01 | 0,02 | 0,02 |
| MgO | 0,03 | 0,02 | 0,04 |
| ZrO₂ | 0,02 | 0,02 | 0,03 |
| BaO | 0,00 | 0,01 | 0,02 |
| Glühverlust | 0,13 | 0,29 | 0,12 |
| Summe | 99,29 | 99,65 | 99,21 |

**Tabelle 7: Materialeigenschaften der Teil- und Endfritten:**

| Fritte | Frittenty p | Brenn-temperatur | WAK (25 [10⁻⁶ - 400°C) 6 K⁻¹] | | Säurelöslichkeit | Biegefestigkeit |
|---|---|---|---|---|---|---|
| | | [°C] | 2x gebrannt | 4x gebrannt | [µg/cm² 1 | [MPa] |
| SD | Leucitfrit te | 750 | 18,3 | 19,5 | - | - |
| HAK105 | Leucitfritte | 820 | 18,4 | 18,1 | - | - |
| HAK108 | Glasfritte | 760 | 8,5 | 8,7 | 5 | - |
| HAK109 | Glasfritte | 770 | 7,3 | 7,2 | 4 | - |
| MU/034/ 18 | Endfritte | 765 | 8,9 | 8,9 | 9 | 112 ± 29 |
| MU/035/ 18 (Vergl.) | Endfritte | 745 | 9,2 | 9,7 | 1 | 101 ± 13 |
| MU/036/ 18 | Endfritte | 760 | 8,6 | 8,7 | 8 | 93 ± 16 |

**Tabelle 8: Verbundfestigkeit der Endfritten zu Lithiumdisilkat- und Zirkondioxid-Gerüsten:**

| Temperaturwechselbeständigkeit auf ZrO₂-Gerüsten | | | |
|---|---|---|---|
| | MU/034/18 | MU/035/18 (Vergl.) | MU/036/18 |
| Sprünge bei 105 °C | keine | keine | keine |
| Sprünge bei 120 °C | keine | keine | keine |
| Sprünge bei 135 °C | keine | ja | keine |
| Sprünge bei 150 °C | keine | ja | ja |
| Sprünge bei 165 °C | ja | keine | ja |
| unbeschädigte Probenkörper | 5/7 | 1/7 | 4/7 |

| Temperaturwechselbeständigkeit auf Lithiumdisilikat-Gerüsten | | | |
|---|---|---|---|
| | MU/034/18 | MU/036/18 | |
| Sprünge bei 105 °C | keine | keine | |
| Sprünge bei 120 °C | keine | keine | |
| Sprünge bei 135 °C | keine | keine | |
| Sprünge bei 150 °C | keine | keine | |
| Sprünge bei 165 °C | keine | keine | |
| unbeschädigte Probenkörper | 7/7 | 7/7 | |

| Ablöse-/Rissbeginn-Prüfung auf kommerziell erhältlichen ZrOz-Gerüsten | | | |
|---|---|---|---|
| Verblendkeramik | Gerüstmaterial | Mittelwert [MPa] | Standardabweichung [MPa] |
| MU/034/18 | VITA YZ^{(R)} T | 44,9 | 4,2 |
| MU/034/18 | VITA YZ^{(R)} XT | 50,8 | 7,8 |
| MU/036/18 | VITA YZ^{(R)} T | 45,5 | 5,9 |
| MU/036/18 | VITA YZ^{(R)} XT | 28,3 | 3,2 |

Die Gerüstmaterialien können von der Firma Vita Zahnfabrik, Deutschland bezogen werden.

### Ergebnis:

Die erfindungsgemäßen Glaskeramiken MU/034/18 und MU/036/18 erfüllen alle gewünschten Voraussetzungen: ausreichend niedrige Brenntemperatur, stabilen WAK bei Mehrfachbrand bei 9 ± 0,5 10⁻⁶ K⁻¹, hohe Biegefestigkeit, niedrige Säurelöslichkeit.

Das Vergleichsbeispiel MU/035/18 ist bei Mehrfachbrand nicht WAK-stabil. Die WAK-Werte nach zweifachem und vierfachem Brand weichen mit 0,5 zu stark voneinander ab (max. 0,3 wäre bei der Endfritte tolerierbar). Weiterhin zeigt das Vergleichsbeispiel eine schlechte Temperaturwechselbeständigkeit. Hier wird ebenfalls ein viermaliger Brand zur Herstellung der verblendeten Gerüste durchgeführt.

## Patentansprüche

1. Glaskeramik zur Verblendung einer dentalen Gerüststruktur, **dadurch gekennzeichnet, dass** die Glaskeramik
60 bis 75 Gew.-% SiO₂,
6 bis 12 Gew.-% B₂O₃,
6 bis 12 Gew.-% K₂O,
Li₂O in einer Menge von weniger als 3 Gew.-% und
Leucit enthält, wobei die Gewichtsprozente jeweils bezogen auf das Gesamtgewicht der Glaskeramik sind.

2. Glaskeramik gemäß Anspruch 1, **dadurch gekennzeichnet, dass** die Glaskeramik einen Erweichungspunkt unterhalb von 790 °C, vorzugsweise unterhalb von 780 °C, besonders bevorzugt von 730 bis 770 °C aufweist, bestimmt gemäß Erhitzungsmikroskop.

3. Glaskeramik gemäß wenigstens einem der vorangehenden Ansprüche, **dadurch gekennzeichnet, dass** die Glaskeramik weiterhin Al₂O₃ in einer Menge von 3 bis 11 Gew.-%, vorzugsweise 5 bis 9 Gew.-% aufweist, bezogen auf das Gesamtgewicht der Glaskeramik.

4. Glaskeramik gemäß wenigstens einem der vorangehenden Ansprüche, **dadurch gekennzeichnet, dass** die Glaskeramik weiterhin Na₂O in einer Menge von 4 bis 11 Gew.-%, vorzugsweise 5 bis 7 Gew.-% aufweist, bezogen auf das Gesamtgewicht der Glaskeramik.

5. Glaskeramik gemäß wenigstens einem der vorangehenden Ansprüche, **dadurch gekennzeichnet, dass** die Glaskeramik weiterhin Li₂O in einer Menge von weniger als 2 Gew.-%, besonders bevorzugt 0,1 bis 1,5 Gew.-% aufweist, bezogen auf das Gesamtgewicht der Glaskeramik.

6. Glaskeramik gemäß wenigstens einem der vorangehenden Ansprüche, **dadurch gekennzeichnet, dass** die Glaskeramik einen Wärmeausdehnungskoeffizienten WAK von weniger als 9,5*10⁻⁶K⁻¹, bevorzugt von 9,3 *10⁻⁶K⁻¹ oder weniger, besonders bevorzugt von 8,3 *10⁻⁶K⁻¹ bis 9,3 *10⁻⁶K⁻¹ oder von 8,3 *10⁻⁶K⁻¹ bis 9,0 *10⁻⁶K⁻¹ aufweist, bestimmt mittels Dilatometer.

7. Glaskeramik gemäß wenigstens einem der vorangehenden Ansprüche, **dadurch gekennzeichnet, dass** die Glaskeramik eine Löslichkeit von weniger als 20 µg/cm², vorzugsweise weniger als 10 µm/cm², besonders bevorzugt 1 bis 5 µg/cm² aufweist, bestimmt gemäß DIN ISO 6872 und/oder die Glaskeramik eine Transluzenz oberhalb von 75 %, vorzugsweise oberhalb von 80%.

8. Glaskeramik gemäß wenigstens einem der vorangehenden Ansprüche, **dadurch gekennzeichnet, dass** die Glaskeramik Leucit in einer Menge von 0,1 bis 10 Gew.-%, besonders bevorzugt in einer Menge von 0,2 bis 5 Gew.-% oder 1 bis 4 Gew.-% aufweist.

9. Verfahren zur Herstellung einer Glaskeramik gemäß wenigstens einem der Ansprüche 1 bis 8, **dadurch gekennzeichnet, dass** das Verfahren die folgenden Schritte umfasst:
a) Herstellen eines Ausgangsglases durch Schmelzen der Grundkomponenten und Abschrecken der Schmelze in Wasser,
b) Aufmahlen des Glases aus Schritt a) unter Erhalt eines Pulvers;
c) Verpressen des Pulvers aus Schritt b) unter Erhalt eines Rohlings;
d) Wärmebehandeln des Rohlings unter Erhalt einer Glaskeramik; und
e) Aufmahlen des Rohlings aus Schritt d) unter Erhalt eines Pulvers.

10. Verwendung einer Glaskeramik gemäß wenigstens einem der Ansprüche 1 bis 8 zur Verblendung einer dentalen Gerüststruktur, vorzugsweise einer keramischen Gerüststruktur auf Basis von Lithiumdisilikat oder ZrO₂, **dadurch gekennzeichnet, dass** die Differenz zwischen dem Wärmeausdehnungskoeffizienten der Verblendstruktur WAK_{VK} und dem Wärmeausdehnungskoeffizienten der Gerüststruktur WAK_{G} nicht mehr als 2,5*10⁻⁶K⁻¹, vorzugsweise weniger als 1,5*10⁻⁶K⁻¹

11. Verwendung gemäß Anspruch 10, **dadurch gekennzeichnet, dass** die Differenz zwischen dem Wärmeausdehnungskoeffizienten der Verblendstruktur WAK_{VK} und dem Wärmeausdehnungskoeffizienten der Gerüststruktur WAK_{G} weniger als 1*10⁻⁶K⁻¹, beträgt, wobei der Wärmeausdehnungskoeffizient jeweils gemäß Dilatometrie bestimmbar ist.

12. Dentale Restauration umfassend eine Gerüststruktur und eine Verblendstruktur, **dadurch gekennzeichnet, dass** es sich bei der Verblendstruktur um eine Glaskeramik nach wenigstens einem der Ansprüche 1 bis 8 handelt.

13. Dentale Restauration gemäß Anspruch 12, **dadurch gekennzeichnet, dass** die Dicke der Verblendstruktur 0,2 bis 3 mm, vorzugsweise 0,5 bis 1,5 mm beträgt.

14. Paste umfassend ein flüssiges Medium und ein Glaskeramikpulver nach wenigstens einem der Ansprüche 1 bis 8 in Form eines Pulvers zur Verblendung einer dentalen Gerüststruktur.

15. Verfahren zur Herstellung einer dentalen Restauration, **dadurch gekennzeichnet, dass** eine Glaskeramik nach wenigstens einem der Ansprüche 1 bis 8 oder eine Paste nach Anspruch 14 auf eine Gerüststruktur aufgebracht wird.

## Claims

1. A glass ceramic for veneering a dental frame structure, **characterized in that** said glass ceramic contains
from 60 to 75% by weight SiO₂,
from 6 to 12% by weight B₂O₃,
from 6 to 12% by weight K₂O,
Li₂O in an amount of less than 3% by weight, and
leucite, wherein said percentages by weight are respectively based on the total weight of the glass ceramic.

2. The glass ceramic according to claim 1, **characterized in that** said glass ceramic has a softening point below 790°C, preferably below 780°C. more preferably from 730 to 770°C, as determined by means of a heating microscope.

3. The glass ceramic according to at least one of the preceding claims, **characterized in that** said glass ceramic further contains Al₂O₃ in an amount of from 3 to 11% by weight, preferably from 5 to 9% by weight, based on the total weight of the glass ceramic.

4. The glass ceramic according to at least one of the preceding claims, **characterized in that** said glass ceramic further contains Na₂O in an amount of from 4 to 11% by weight, preferably from 5 to 7% by weight, based on the total weight of the glass ceramic.

5. The glass ceramic according to at least one of the preceding claims, **characterized in that** said glass ceramic further contains Li₂O in an amount of less than 2% by weight, more preferably from 0.1 to 1.5% by weight, based on the total weight of the glass ceramic.

6. The glass ceramic according to at least one of the preceding claims, **characterized in that** said glass ceramic has a coefficient of thermal expansion CTE of less than 9.5*10⁻⁶K⁻¹, preferably from 9.3*10⁻⁶K⁻¹ or less, more preferably from 8.3*10⁻⁶K⁻¹ to 9.3*10⁻⁶K⁻¹, or from 8.3*10⁻⁶K⁻¹ to 9.0*10⁻⁶K⁻¹, as determined by means of a dilatometer.

7. The glass ceramic according to at least one of the preceding claims, **characterized in that** said glass ceramic has a solubility of less than 20 µg/cm², preferably less than 10 µm/cm², more preferably 1 to 5 µg/cm², as determined according to DIN ISO 6872, and/or said glass ceramic has a translucency of above 75%, preferably above 80%.

8. The glass ceramic according to at least one of the preceding claims, **characterized in that** said glass ceramic contains leucite in an amount of 0.1 to 10% by weight, more preferably in an amount of 0.2 to 5% by weight, or 1 to 4% by weight.

9. A process for preparing a glass ceramic according to at least one of claims 1 to 8, **characterized in that** said process comprises the following steps:
a) preparing a starting glass by melting the basic components, and quenching the melt in water,
b) grinding the glass from step a) to obtain a powder;
c) pressing the powder from step b) to obtain a blank;
d) heat-treating the blank to obtain a glass ceramic; and
e) grinding the blank from step d) to obtain a powder.

10. Use of a glass ceramic according to at least one of claims 1 to 8 for veneering a dental frame structure, preferably a ceramic frame structure based on lithium disilicate or ZrO₂, **characterized in that** the difference between the coefficient of thermal expansion of the veneering structure, CTEvs, and the coefficient of thermal expansion of the frame structure, CTE_{F}, is not more than 2.5*10⁻⁶K⁻¹, preferably less than 1.5*10⁻⁶K⁻¹.

11. Use according to claim 10, **characterized in that** the difference between the coefficient of thermal expansion of the veneering structure, CTEvs, and the coefficient of thermal expansion of the frame structure, CTE_{F}, is less than 1*10⁻⁶K⁻¹, wherein the coefficient of thermal expansion can respectively be determined by dilatometry.

12. A dental restoration, comprising a frame structure and a veneering structure, **characterized in that** said veneering structure is a glass ceramic according to at least one of claims 1 to 8.

13. The dental restoration according to claim 12, **characterized in that** the thickness of the veneering structure is from 0.2 to 3 mm, preferably from 0.5 to 1.5 mm.

14. A paste, comprising a liquid medium and a glass ceramic powder according to at least one of claims 1 to 8 in the form of a powder for veneering a dental frame structure.

15. A process for producing a dental restoration, **characterized in that** a glass ceramic according to at least one of claims 1 to 8 or a paste according to claim 14 is applied to a frame structure.

## Revendications

1. Vitrocéramique pour mettre des facettes sur une structure d'échafaudage dentaire, **caractérisée en ce que** la vitrocéramique contient
de 60 à 75 % en poids SiO₂,
de 6 à 12 % en poids B₂O₃,
de 6 à 12 % en poids K₂O,
Li₂O dans une quantité de moins de 3 % en poids, et
leucite, dans laquelle les pourcentages en poids sont respectivement par rapport au poids total de la vitrocéramique.

2. Vitrocéramique selon la revendication 1, **caractérisée en ce que** ladite vitrocéramique présente un point de ramollissement inférieur à 790°C, de préférence inférieur à 780°C, de manière particulièrement préférée de 730 à 770°C, déterminé au microscope chauffant.

3. Vitrocéramique selon au moins une des revendications précédentes, **caractérisée en ce que** ladite vitrocéramique contient en outre du Al₂O₃ en une teneur de 3 à 11 % en poids, de préférence de 5 à 9 % en poids, par rapport au poids total de la vitrocéramique.

4. Vitrocéramique selon au moins une des revendications précédentes, **caractérisée en ce que** ladite vitrocéramique contient en outre du Na₂O en une teneur de 4 à 11 % en poids, de préférence de 5 à 7 % en poids, par rapport au poids total de la vitrocéramique.

5. Vitrocéramique selon au moins une des revendications précédentes, **caractérisée en ce que** ladite vitrocéramique contient en outre du Li₂O en une teneur inférieure à 2 % en poids, de manière particulièrement préférée de 0,1 à 1,5 % en poids, par rapport au poids total de la vitrocéramique.

6. Vitrocéramique selon au moins une des revendications précédentes, **caractérisée en ce que** ladite vitrocéramique présente un coefficient de dilatation thermique, CDT, inférieur à 9,5*10⁻⁶K⁻¹, de préférence 9,3*10⁻⁶K⁻¹ ou moins, de préférence encore de 8,3 *10⁻⁶K⁻¹ à 9,3 *10⁻⁶K⁻¹ ou de 8,3 *10⁻⁶K⁻¹ à 9,0 *10⁻⁶K⁻¹, déterminé au dilatomètre.

7. Vitrocéramique selon au moins une des revendications précédentes, **caractérisée en ce que** ladite vitrocéramique présente une solubilité inférieure à 20 µg/cm², de préférence inférieure à 10 µm/cm², de manière particulièrement préférée de 1 à 5 µg/cm², déterminée selon DIN ISO 6872, et/ou ladite vitrocéramique présente une translucidité supérieure à 75 %, de préférence supérieure à 80 %.

8. Vitrocéramique selon au moins une des revendications précédentes, **caractérisée en ce que** la vitrocéramique contient de la leucite dans une quantité de 0,1 à 10 % en poids, de préférence dans une quantité de 0,2 à 5 % en poids, ou 1 à 4 % en poids.

9. Procédé pour préparer une vitrocéramique selon au moins une des revendications 1 à 8, **caractérisé en ce que** le procédé comprend les étapes suivantes consistant à :
a) préparer un verre de départ en fondant les composants de base et trempage de la masse fondue dans de l'eau,
b) moudre le verre obtenu dans l'étape a) pour obtenir une poudre,
c) presser la poudre obtenue dans l'étape b) pour obtenir un corps vert,
d) traiter le corps vert à chaud pour obtenir une vitrocéramique, et
e) moudre le corps vert obtenu dans l'étape d) pour obtenir une poudre.

10. Utilisation d'une vitrocéramique selon au moins une des revendications 1 à 8 pour mettre des facettes sur une structure d'échafaudage dentaire, de préférence sur une structure d'échafaudage céramique à base de disilicate de lithium ou ZrO₂, **caractérisée en ce que** la différence entre le coefficient de dilatation thermique de la structure de facette, CDT_{VK}, et le coefficient de dilatation thermique de la structure d'échafaudage, CDT_{G}, n'excède pas 2,5*10⁻⁶K⁻¹, de préférence est moins de 1,5*10⁻⁶K⁻¹.

11. Utilisation selon la revendication 10, **caractérisée en ce que** la différence entre le coefficient de dilatation thermique de la structure de facette, CDT_{VK}, et le coefficient de dilatation thermique de la structure d'échafaudage, CDT_{G}, est moins de 1*10⁻⁶K⁻¹, le coefficient de dilatation thermique chaque fois pouvant être déterminé par dilatométrie.

12. Restauration dentaire comprenant une structure d'échafaudage et une structure de facette, **caractérisée en ce que** ladite structure de facette est une vitrocéramique selon au moins une des revendications 1 à 8.

13. Restauration dentaire selon la revendication 12, **caractérisée en ce que** l'épaisseur de la structure de facette est de 0,2 à 3 mm, de préférence de 0,5 à 1,5 mm.

14. Pâte comprenant un milieu liquide et une poudre de vitrocéramique selon au moins une des revendications 1 à 8 sous la forme d'une poudre pour mettre des facettes sur une structure d'échafaudage dentaire.

15. Procédé pour préparer une restauration dentaire, **caractérisé en ce qu'**une vitrocéramique selon au moins une des revendications 1 à 8 ou une pâte selon la revendication 14 est appliquée à une structure d'échafaudage.
